# EUROPEAN PATENT APPLICATION

(11) **EP 3 991 700 A1**
(43) Date of publication of application: **04.05.2022**
(21) Application number: 20831540.8
(22) Date of filing: 23.06.2020
(51) Int. Cl.: A61F 2/91, A61B 18/12

(54) **ATRIAL SEPTOSTOMY DEVICE**

(30) Priority: 25.06.2019 CN 201910557780; 25.06.2019 CN 201920966952 U
(71) Applicant: Hangzhou Noya Medtech Co., Ltd., Hangzhou, Zhejiang 310000 (CN)
(72) Inventor: WANG, Yongsheng, Hangzhou, Zhejiang 310000 (CN); DONG, Yuanbo, Hangzhou, Zhejiang 310000 (CN)
(74) Representative: Krauns, Christian
(86) International application number: PCT/CN2020/097681
(87) International publication number: WO 2020/259492

(57) **Abstract**

An atrial septostomy device (20) includes a support frame (21) for propping a puncture hole in the atrial septum. The support frame (21) includes an expansion portion (23) for propping the puncture hole, a connecting portion (27) connected to a proximal end of the expansion portion (23), and an extension portion (25) connected to a distal end of the expansion portion (23). A distal end of the extension portion (25) has a converged portion (250) that is parallel to the axis of the support frame (21) or extends towards the axis of the support frame (21). The support frame (21) is provided with an ablation electrode (60) for ablating tissues surrounding the puncture hole. The opening shape after treatment with the atrial septostomy device (20) is regular, unlikely to be blocked, and able to be kept unobstructed. Since the distal end of the extension portion (25) has a converged portion that is parallel to the axis of the support frame (21) or extends towards the axis of the support frame (21), the extension portion (25) as a free end is prevented from damaging the myocardial tissue when the atrial septostomy device (20) enters the heart tissue, so as to improve the safety.

## Description

### TECHNICAL FIELD

The present invention relates to the technical field of interventional medical devices, and particularly to an atrial septostomy device for transcutaneous interventional therapy.

### BACKGROUND

Heart failure is a complex clinical syndrome in which the ventricular filling or pumping capacity is impaired due to any structural and/or functional abnormality of the heart. The main clinical manifestations of heart failure include dyspnea, fatigue (reduced exercise tolerance) and fluid retention (pulmonary congestion and peripheral edema). Heart failure is the severe end stage of various heart diseases, and has high incidence, thus being one of the most notorious cardiovascular diseases. Based on the location of heart failure, it can be divided into left-sided, right-sided and whole heart failure.

Heart failure is a serious disease with high incidence and fatality rate. The incidence of heart failure is 2-3%, and over 12 million patients suffer from heart failure in China. The main causes of heart failure include high blood pressure, coronary heart disease, myocardial infarction, heart valve diseases, atrial fibrillation, and myocardiopathy. Cardiovascular disease causes damage to the left ventricle, leading to the pathological remodeling of the left ventricle, and a decrease in heart function. Every time a patient with myocardial infarction survived after treatment, a potential patient with heart failure comes.

Therapeutically, after optimizing the drug treatment, the symptoms of the patient still reoccur. The current drug treatment almost has a good therapeutic effect only on patients with reduced ejection fraction; and for those with preserved ejection fraction, the therapeutic effect is undesirable. Cardiac resynchronization therapy is not suitable for all patients with heart failure, and more than 20% of patients fail to make pace in cardiac resynchronization therapy. The surgery with a left ventricular assist device requires extracorporeal circulation, causes a major trauma, and has a high incidence of complications, and the left ventricular assist device itself is expensive and difficult to be obtained. Heart transplantation is the ultimate solution; however, the source of donors is very limited, and the cost is high.

Atrial septostomy is to create an opening in a patient's atrial septum, to form a shunt between the left and right heart atria. The atrial septostomy is useful in the treatment of pulmonary hypertension (right-to-left shunt) or left-sided heart failure (left-to-right shunt), and clinically proven to be effective.

The typical atrial septostomy, for example, balloon atrial septostomy, has the problem of tendency to rebound of the myocardial tissue after the septostomy, and the opening will get narrowed or even be closed completely after a period of time. To solve the problem of narrowed or even closed opening, an opening stent is provided in the prior art, which discloses an implant for atrial shunt, wherein after transseptal puncture, a shunt device is implanted at the atrial septal puncture site by transcutaneous delivering an implant, to keep the shunt opening unobstructed.

Another opening instrument is disclosed, which includes a cutting device and a grabbing device. When the instrument is used to make an opening in the tissue, the grasping device locates and grasps a part of the tissue that needs to be cut, then a cutter of the cutting device cuts the part of the tissue grabbed by the grabbing device, and to form an opening, wherein the part of tissue cut is taken out of the body by the grasping device.

The above technology has the following shortcomings. In the implant for atrial shunt, a device will be left in the body at the opening, which may easily cause thrombosis or falling off of the instrument, to form an embolism. Moreover, due to the growth and adherence of endothelial cells, the opening of the device may be blocked, so the channel is closed and loses the shunting effect. In addition, during the operation, the heart tissue is cut mechanically or by a high-frequency electric knife at high risk. For example, when the grasping device is loosened or withdrawn during the operation, the cut tissue may be caused to fall off to form an embolism. Furthermore, during the cutting process, the loosening of the grasping device can easily cause damage to other myocardial tissues.

### SUMMARY

An object of the present invention is to provide an atrial septostomy device where the opening is unlikely to be blocked and no damage is caused to other myocardial tissues.

To solve the above technical problems, the present invention provides an atrial septostomy device, which includes a support frame for propping a puncture hole on an atrial septum, wherein the support frame includes an expansion portion for expanding the puncture hole, a connecting portion connected to a proximal end of the expansion portion, and an extension portion connected to a distal end of the expansion portion. A distal end of the extension portion has a converged portion that is parallel to the axis of the support frame or extends in a radial direction towards the axis of the support frame. The support frame is provided with at least one ablation electrode for ablating tissues surrounding the puncture hole.

The atrial septostomy device in an atrial septostomy system of the present invention includes a support frame that expands the puncture hole in the atrial septum, and an ablation electrode provided on the support frame. The ablation electrode is in contact with the atrial septal tissue around the puncture hole, and the ablation electrode receives a radio frequency power to ablate the tissue of the atrial septum at the puncture hole, so as to inactivate the atrial septal tissue around the puncture hole, prevent the puncture hole from being blocked by the grown and adhered endothelial cells during tissue repair, and keep the shape of the opening stable after the opening is made by the atrial septostomy system. Therefore, the opening shape after treatment with the atrial septostomy device is regular, unlikely to be blocked, and able to be kept unobstructed, so that the blood flow can be shunted smoothly between the left and right heart chambers. In addition, a distal end of an extension portion of the atrial septostomy device has a converged portion that is parallel to the axis of the support frame or extends towards the axis of the support frame. That is, the converged portion converges towards the axis. This prevents the extension portion as a free end from damaging the myocardial tissue when the atrial septostomy device enters the heart tissue, so as to improve the safety.

### BRIEF DESCRIPTION OF THE DRAWINGS

To describe the technical solutions according to the embodiments of the present application more clearly and fully, the drawings needed to be used in the embodiments of the present application will be described briefly below. Apparently, the drawings in the following description only show some embodiments of the present application. Other obvious variations can be obtained by persons of ordinary skill in the art based on these drawings without creative efforts.
FIG. 1 is a schematic structural view of an atrial septostomy system according to a first embodiment of the present application.
FIG. 2 is a schematic structural perspective view of an atrial septostomy device in the atrial septostomy system shown in FIG. 1.
FIG. 3 is a schematic side view of the atrial septostomy device shown in FIG. 2.
FIG. 4 is a schematic structural view of an atrial septostomy device according to a second embodiment of the present application.
FIG. 5 is a schematic structural view of an atrial septostomy device according to a third embodiment of the present application.
FIG. 6 is a schematic structural view of an atrial septostomy device according to a fourth embodiment of the present application.
FIG. 7 is a schematic structural view of an atrial septostomy device according to a fifth embodiment of the present application.
FIG. 8 is a schematic structural view of an atrial septostomy device according to a sixth embodiment of the present application.
FIG. 9 is a schematic structural view of an atrial septostomy device according to a seventh embodiment of the present application.
FIG. 10 is a schematic structural view of an atrial septostomy device according to an eighth embodiment of the present application.
FIG. 11 is a schematic structural view of an atrial septostomy device according to a ninth embodiment of the present application.
FIG. 12 is a schematic structural view of an atrial septostomy device according to a tenth embodiment of the present application.
FIG. 13 is a schematic structural perspective view of an atrial septostomy device according to an eleventh embodiment of the present application.
FIG. 14 is a schematic side view of the atrial septostomy device shown in FIG. 13.
FIG. 15 is a schematic structural view of an atrial septostomy system according to a twelfth embodiment of the present application.
FIG. 16 is a schematic structural view of an atrial septostomy device shown in FIG. 15.
FIG. 17 is a schematic structural perspective view of an atrial septostomy device according to a thirteenth embodiment of the present application.
FIG. 18 is a schematic side view of the atrial septostomy device shown in FIG. 17.
FIG. 19 is a schematic structural perspective view of an atrial septostomy device according to a fourteenth embodiment of the present application.
FIG. 20 is a schematic structural perspective view of an atrial septostomy device according to a fifteenth embodiment of the present application.

### DESCRIPTION OF THE EMBODIMENTS

The technical solutions according to the embodiments of the present application will be described clearly and fully in combination with the accompanying drawings in the embodiments of the present application. Apparently, the embodiments described are merely some, but not all of the embodiments of the present application. All other embodiments obtained by persons of ordinary skill in the art based on the embodiments of the present application without creative efforts shall fall within the protection scope claimed by the present application.

In addition, the following description of various embodiments is provided to exemplify the specific embodiments of the present application with reference to accompanying drawings. The directional terms mentioned in the present application, such as, "up", "down", "front", "back", "left", "right", "inner", "outer", and "side", etc., are only given with reference to the direction of the drawings. Therefore, the directional terms used are to better and more clearly explain and understand the present application, instead of indicating or implying that the device or element referred to needs to have a specific orientation, and be constructed and operated in a specific orientation. Therefore, they cannot be understood as a restriction on the present application. In addition, "axial" direction means the direction of the axis of the pushing member or the direction of the axis of the pushing tube.

Definition of orientations: For the sake of clear description, the end close to the operator during the operation is called "proximal" end and the end far away from the operator is called "distal" end. Axial direction refers to a direction parallel to the connection line between a distal center and a proximal center of an instrument. The above definitions are merely for convenience only and shall not be construed as limiting the present application.

In this embodiment, the mechanism of the pushing device in the interventional medical instrument according to the present application and use thereof are specifically illustrated with an example in which the interventional medical device is an occluder for occluding rupture of aortic dissection. However, the scope of application of the pushing device in the interventional medical device is not limited to the occluder.

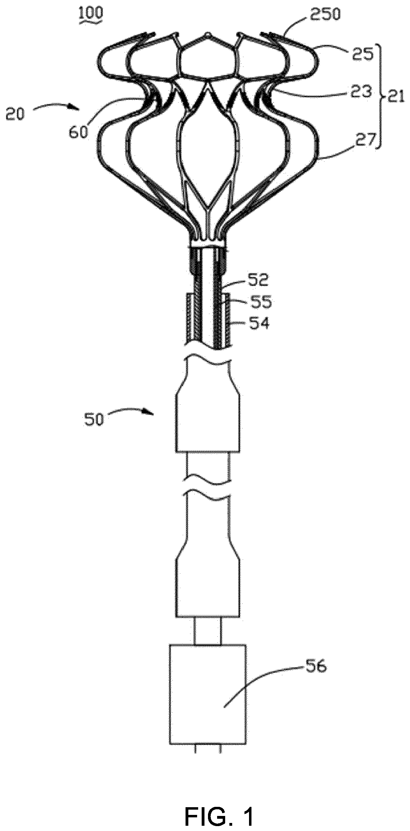

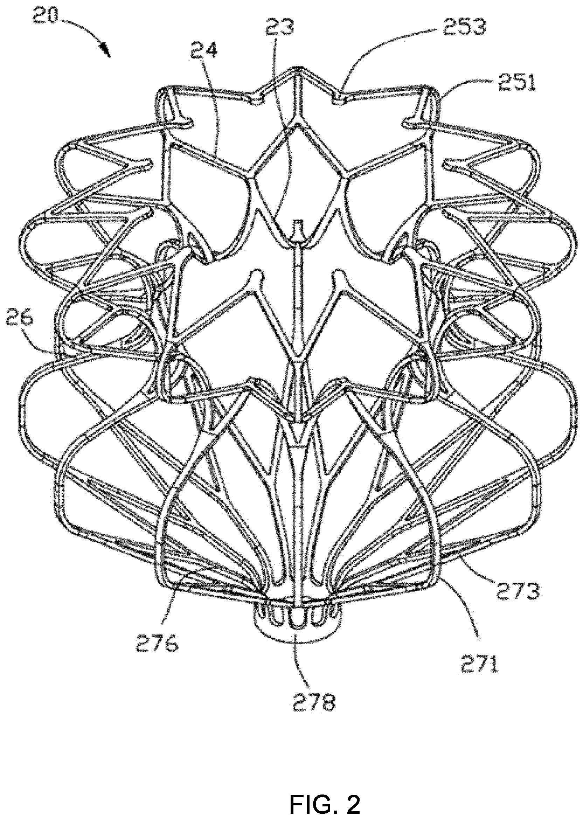

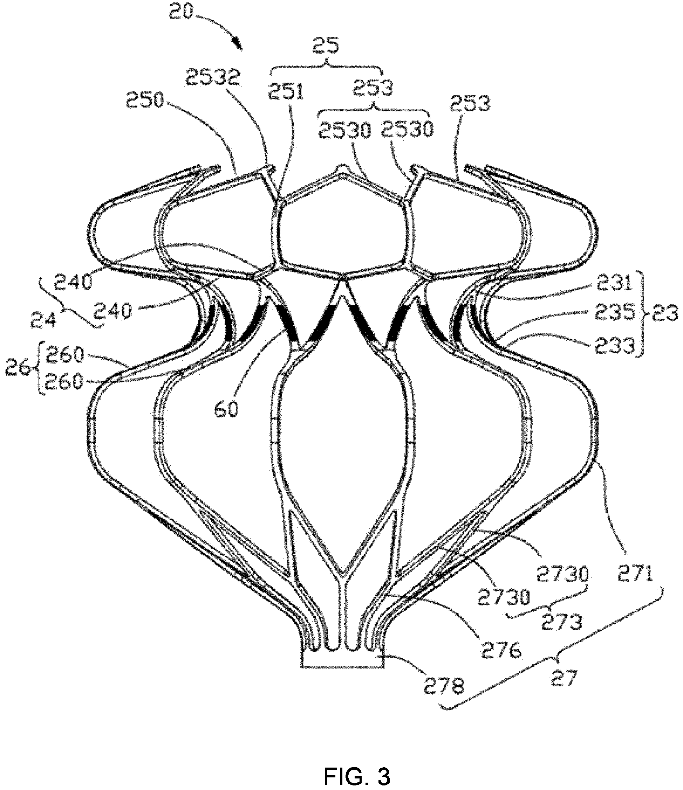

Referring to FIGs. 1 to 3, FIG. 1 is a schematic structural view of an atrial septostomy system according to a first embodiment of the present application, FIG. 2 is a schematic structural perspective view of an atrial septostomy device in the atrial septostomy system shown in FIG. 1, and FIG. 3 is a schematic side view of the atrial septostomy device shown in FIG. 2. The present invention provides an atrial septostomy system 100, which includes an atrial septostomy device 20 and a delivery mechanism 50 for delivering the atrial septostomy device 20. The atrial septostomy device 20 includes a support frame 21 for expanding a puncture hole in an atrial septum. The support frame 21 includes an expansion portion 23 for expanding the puncture hole, an extension portion 25 connected to a distal end of the expansion portion 23, and a connecting portion 27 connected to a proximal end of the expansion portion 23. A distal end of the extension portion 25 has a converged portion 250 that converges towards the axis of the support frame 21. The support frame 21 is provided with at least one ablation electrode 60 for ablating the tissue surrounding the puncture hole. The delivery mechanism 50 is used to deliver the atrial septostomy device 20 into the puncture hole in the atrial septum, and the ablation electrode 60 is connected to a radio frequency power supply through the delivery mechanism 50. The ablation electrode 60 is attached to and in contact with the tissues at and around the puncture hole, and the ablation electrode 60 is electrically connected to the radio frequency power supply. The ablation electrode 60 receives the energy output from the radio frequency power supply to ablate the tissue around the puncture hole in the atrial septum.

In the atrial septostomy device 20 of the atrial septostomy system 100 of the present invention, the support frame 21 for expanding the puncture hole in the atrial septum, and the ablation electrode 60 arranged on the support frame 21 are provided. The ablation electrode 60 is in contact with the atrial septal tissue around the puncture hole, and the ablation electrode 60 receives a radio frequency power to ablate the tissue around the puncture hole in the atrial septum, so as to inactivate the atrial septal tissue around the puncture hole, prevent the puncture hole from being blocked by the grown and adhered endothelial cells during tissue repair, and maintain the shape of the opening stable after the opening is made by the atrial septostomy system 100. Therefore, the opening shape after treatment with the atrial septostomy device 20 is regular, unlikely to be blocked, and able to be kept unobstructed, so that the blood flow can be shunted smoothly between the left and right heart chambers. In addition, at the distal end of the extension portion 25 of the atrial septostomy device 20, the converged portion 250 that extends towards the axis of the support frame 21 is provided. That is, the converged portion 250 converges towards the axis. This prevents the extension portion 25 as a free end from damaging the myocardial tissue when the atrial septostomy device 20 enters the heart tissue, so as to improve the safety.

As shown in FIGs. 2 and 3, the support frame 21 is a self-expandable opening device, and the support frame 21 may be an elastic metal support frame or an elastic non-metal support frame. In this embodiment, the support frame 21 is a nickel alloy stent. When the atrial septostomy device 20 is delivered by a sheath, the support frame 21 can be compressed into a lower profile for delivery in the sheath. When the atrial septostomy device 20 is released in the heart, the support frame 21 can automatically expand to a required shape and size, so that the support frame 21 can expand the puncture hole in the atrial septum to form an opening. That is, the expansion portion 23 of the support frame 21 provides, in the puncture hole, a radial support for the inner wall of the puncture hole.

The support frame 21 can be formed by cutting an elastic tube, and the support frame 21 has a cylindrical frame structure after being released in the body to keep the puncture hole in the atrial septum unblocked. The support frame 21 may also be woven with a nickel alloy wire, or partially formed by weaving a wire and partially formed by cutting a tube, wherein the various different portions can be welded or fixed to each other by a connecting member. The elastic tube is made of a shape memory metal material, and preferably Nitinol. The support frame 21 may generally have, but not limit to, a straight cylindrical shape, a disc shape, a conical shape and other suitable shapes. In this embodiment, the support frame 21 is substantially 8-shaped after being completely released.

As shown in FIG. 3, after the atrial septostomy device 20 is completely released, the expansion portion 23 of the support frame 21 is cylindrical. The expansion portion 23 and the extension portion 25 are connected by a first positioning portion 24; and the expansion portion 23 and the connecting portion 27 are connected by a second positioning portion 26. When the atrial septostomy device 20 is implanted into the puncture hole in the atrial septum, the expansion portion 23 props the inner wall of the puncture hole, and the first positioning portion 24 and the second positioning portion 26 are respectively positioned at two opposite sides of the atrial septum. Specifically, the first positioning portion 24 is positioned at a distal side of the atrial septum, and the second positioning portion 26 is positioned at a proximal side of the atrial septum. The diameter of the first positioning portion 24 is greater than the diameter of the expansion portion 23, and the first positioning portion 24 is provided with a positioning surface, a positioning line or a positioning point that contacts the atrial septum. Specifically, a side of the first positioning portion 24 facing the expansion portion 23 is provided with a positioning surface, a positioning line or a positioning point that can press against the atrial septal tissue. The positioning surface, positioning line or positioning point abuts against the atrial septal tissue, to prevent the atrial septostomy device 20 from moving proximally. The ablation electrode 60 can be provided on the positioning point, positioning line or positioning surface.

The diameter of the second positioning portion 26 is greater than the diameter of the expansion portion 23, and the second positioning portion 26 is provided with a positioning surface, a positioning line or a positioning point that contacts the atrial septum. Specifically, a side of the second positioning portion 26 facing the expansion portion 23 is provided with a positioning surface, a positioning line or a positioning point that can press against the atrial septal tissue. The positioning surface, positioning line or positioning point abuts against the atrial septal tissue, to prevent the atrial septostomy device 20 from moving distally. As a result, the atrial septostomy device 20 is positioned on the atrial septum. The ablation electrode 60 can be provided on the positioning point, positioning line or positioning surface.

In other embodiments, the ablation electrodes 60 can be separately provided or concurrently provided on the positioning surfaces, positioning lines or positioning points of the first positioning portion 24 and the second positioning portion 26. In this embodiment, the expansion portion 23 has a wave-shaped annular structure arranged continuously in the circumferential direction for at least one circle, wherein the first positioning portion 24 is connected to peaks of the wave-shaped annular structure and the second positioning portion 26 is connected to valleys of the wave-shaped annular structure. Specifically, the expansion portion 23 is formed by a plurality of V-shaped support rods connected end to end in sequence to form the wave-shaped annular structure. The wave-shaped annular structure includes peaks 231, valleys 233 and bars 235, wherein circumferentially adjacent bars 235 are connected at a distal end to form the peaks 231, circumferentially adjacent bars 235 are connected at a proximal end to form the valleys 233, and a middle portion of each bar 235 is arc shaped by recessing towards the axis of the support frame 20. A proximal end of the first positioning portion 24 is connected to the peaks 231, and a distal end of the second positioning portion 26 is connected to the valleys 233. The expansion portion 23 is generally required to be easy for radial compression and to maintain the necessary strength. When the expansion portion 23 is released in the puncture hole in the atrial septum, the expansion portion 23 can automatically expand to a required shape and size, and produce a radial support for the tissue at an inner surface of the puncture hole in contact with it.

At least one marker member is provided on the expansion portion 23 and the marker member is fixed by inlay, hot pressing, or other methods. Specifically, a plurality of marker members are provided on one of the peaks 231, the valleys 233 and the bars 235 of the expansion portion 23, to form a circle of marker members on the expansion portion 23. Alternatively, the marker members may be provided on two of the peaks 231, the valleys 233 and the bars 235, to form two circles of marker members at an interval on the expansion portion 23. Alternatively, marker members are provided on the peaks 231, the valleys 233 and the bars 235, to form three circles of marker members at intervals on the expansion portion 23. This facilitates the location of the expansion portion 23 in the puncture hole in the atrial septum. The marker member can be made of gold, platinum, tantalum or other materials.

In other embodiments, at least one circle of flexible marker wire is provided on the expansion portion 23, and the marker wire is fixed by winding, inlay, hot pressing, or other methods.

In other embodiments, the expansion portion 23 may also be formed by a plurality of X-shaped support rods connected in sequence to form the annular structure. The first positioning portion 24 is connected to distal ends of the X-shaped support rods, and the second positioning portion 26 is connected to proximal ends of the X-shaped support rods.

In other embodiments, the expansion portion 23 may also be an annular structure woven with a nickel alloy wire to have a meshed annular structure. The first positioning portion 24 is connected to a distal end of the meshed annular structure, and the second positioning portion 26 is connected to a proximal end of the meshed annular structure.

The extension portion 25 includes a plurality of first connecting rods 251 provided at a distal end of the first positioning portion 24, and a plurality of extension members 253 each provided at a distal end of a respective first connecting rod 251. The plurality of first connecting rods 251 are arranged in a circle along the circumferential direction of the first positioning portion 24. A proximal end of each first connecting rod 251 is connected to the first positioning portion 24, and the distal end of the first connecting rod 251 is connected to a corresponding extension member 253. A middle portion of the first connecting rod 251 protrudes away from the axis of the support frame 20 to form an arc-shaped rod. The plurality of extension members 253 together form the converged portion 250.

Each extension member 253 extends inclinedly from the distal end of a corresponding first connecting rod 251 towards the axis of the support frame 20, and the plurality of extension members 253 are annularly arranged in a circle about the axis of the support frame 20 to form the converged portion 250.

A distal end of each extension member 253 is closer to the axis of the support frame 20 than the corresponding first connecting rod 251, and the distal end of the extension member 253 is smoothed. Specifically, an outer peripheral surface of the distal end of the extension member 253 is designed as an arc surface or have a rounded corner, or the distal end of the extension member 253 is designed as a circular sheet, a spherical structure or a spheroid structure.

In this embodiment, each extension member 253 includes two branch rods 2530 bent and extending radially inwards from two sides from the distal end of the first connecting rod 251 towards the axis of the support frame 20. Distal ends of two approaching branch rods 2530 of each two adjacent extension members 253 intersect to form an intersected portion 2532. A distal end of the intersected portion 2532 is closer to the axis of the support frame 20 than the distal end of the first connecting rod 251.

The distal end of each intersected portion 2532 is smoothed. Specifically, an outer peripheral surface of the distal end of the intersected portion 2532 is designed as an arc surface or have a rounded corner, or the intersected portion 2532 has a round structure or a spheroid structure.

The distal end of the extension member 253 is smoothed, to prevent the extension member 253 as a free end from damaging the myocardial tissue when the atrial septostomy device 20 enters the heart tissue, so as to improve the safety.

The first positioning portion 24 includes a plurality of pairs of first positioning rods 240, and the first positioning rods 240 of each pair are bent and extend radially from two sides of a corresponding peak 231 of the expansion portion 23 in a direction away from the axis of the support frame 20. Distal ends of two approaching first positioning rods 240 respectively extending from two adjacent peaks 231 intersect. The plurality of first connecting rods 251 respectively correspond to the plurality of distal intersections of the first positioning portion 24. That is, the proximal end of each first connecting rod 251 is connected to the corresponding distal intersection of the first positioning portion 24.

In other embodiments, at least one circle of marker members are provided on the first positioning portion 24, and the marker members are fixed by inlay, hot pressing, or other methods. Specifically, each first positioning rod 240 of the first positioning portion 24 is provided with a marker member by inlay or hot pressing. Alternatively, the first positioning portion 24 is provided with at least one circle of flexible marker wire, and the flexible marker wire is fixed by winding, inlay, hot pressing, or other methods.

The second positioning portion 26 includes a plurality of second positioning rods 260 each extending from a respective valley 233 of the expansion portion 23 in a direction away from the axis of the support frame 20. Specifically, a distal end of each second positioning rod 260 is connected to the corresponding valley 233, and a proximal end extends inclinedly proximally in a direction away from the axis of the support frame 20. In other embodiments, at least one circle of marker members are provided on the second positioning portion 26, and the marker members are fixed by inlay, hot pressing, or other methods. Specifically, each second positioning rod 260 of the second positioning portion 26 is provided with a marker member by inlay or hot pressing. Alternatively, the second positioning portion 24 is provided with at least one circle of flexible marker wire, and the flexible marker wire is fixed by winding, inlay, hot pressing, or other methods.

A proximal end of the connecting portion 27 is connected to the delivery mechanism 50, and a distal end of the connecting portion 27 is connected to the second positioning portion 26. The connecting portion 27 includes a plurality of second connecting rods 271 provided at the proximal end of the second positioning portion 26, a plurality of support members 273 each provided at a proximal end of a respective second connecting rod 271, a plurality of extension rods 276 each provided at a proximal end of a respective support member 273, and a connector 278 provided at proximal ends of the extension rods 276. The plurality of second connecting rods 271 are arranged along the circumferential direction of the support frame 20, wherein a distal end of each second connecting rod 271 is connected to the proximal end of a corresponding second positioning rod 260, a proximal end of each second connecting rod 271 is connected to a corresponding support member 273, and a middle portion of each second connecting rod 271 protrudes away from the axis of the support frame 20 to form an arc-shaped rod. Each support member 273 includes two branch support rods 2730 bent and extending radially inwardly from two sides of the proximal end of the corresponding second connecting rod 271 towards the axis of the support frame 20. Proximal ends of two approaching branch support rods 2730 of each two adjacent support members 273 intersect to form an intersected portion. A distal end of the intersected portion is closer to the axis of the support frame 20 than the distal end of the second connecting rod 271. A proximal end of each intersected portion further extends proximally to form the extension rod 276. Proximal ends of the extension rods 276 converge at the connector 278, to form a substantially cage shaped structure. The connector 278 has a cylindrical or elliptical tubular structure, with an axial length about 1 to 3 mm, and a smooth edge without sharp corners. The connector 278 can be fixedly or detachably connected to the delivery mechanism 50.

In this embodiment, the expansion portion 23 is provided with the ablation electrodes 60. Specifically, each bar 235 is provided with an ablation electrode 60 on a side away from the axis of the support frame 20, and these ablation electrodes 60 are arranged in a circle along the circumference of the expansion portion 23, with a small gap between two adjacent ablation electrodes 60. In other embodiments, every other bar 235 is provided with an ablation electrode 60 on a side away from the axis of the support frame 20, and these ablation electrodes 60 are arranged in a circle along the circumference of the expansion portion 23, with a large gap between two adjacent ablation electrodes 60. When the ablation electrode 60 is released at the puncture hole in the atrial septal tissue, the ablation electrode 60 can ablate the tissue at the puncture hole, to prevent the rebound of the tissue at the puncture hole, and better maintain the shape of the opening.

When the support frame 21 is made of a conductive material, parts of the support frame 21 can directly serve as the ablation electrodes 60. The ablation electrodes 60 can be provided at a position on the expansion portion 23, the first positioning portion 24, or the second positioning portion 26 that contacts tissue surrounding the puncture hole. Because the support frame 21 itself is made of a conductive material, insulation treatment is needed on the outer surface at portions of the support frame 21 other than the parts used as the ablation electrode 60, to prevent the electric conduction with blood due to the contact of the remaining portions of the outer surface which results in reducing the impedance and causing it impossible to complete the ablation of the atrial septal tissue at a specific location. The insulation treatment includes an insulating coating on the outer surface of the support frame or an insulating sleeve mounted around the support frame. Because the support frame 21 itself is conductive, it can be directly connected to a radio frequency power supply by the connector 278, such that the radio frequency energy is transferred by the support frame 21 to the ablation electrodes 60 that contact the tissue surrounding the puncture hole. In this embodiment, to further concentrate the energy on the expansion portion 23 to ablate the atrial septal tissue, an insulating coating can be applied to the outer surface at other portions of the support frame 21 that is in pressure contact with the atrial septal tissue. Further, the insulating coating used is a plated Parylene insulating coating.

Further, the outer surface of the support frame as the ablation electrodes 60 is provided with a gold-plated layer or a platinum-plated layer. These metal coatings can not only improve the conductivity of the electrode, but also be used as imaging marks, making it easier for an operator to observe the positions of the electrodes. The gold-plated layer or the platinum-plated layer may also be replaced by other plating materials with good conductivity and imaging performance.

In another embodiment, the ablation electrode 60 may be a wire electrode provided on the expansion portion 23, and the electrode wire is electrically connected to a radio frequency power supply by an external wire. To concentrate the radio frequency energy on the electrode wire, insulation treatment can be carried out at a position where the electrode wire contacts the expansion portion 23, by coating an insulating layer or wrapping an insulating film or an insulating sleeve.

As shown in FIG. 1, the delivery mechanism 50 includes a loader, a pushing member 52, a sheath 54, a core 55, a conductive pusher 56 and an ablation power source. The pushing member 52 is detachably or integrally fixedly connected to the atrial septostomy device 20. A guidewire is provided in the pushing member 52, wherein one end of the wire is electrically connected to the ablation electrode 60 of the atrial septostomy device 20; and the other end of the wire is electrically connected to the ablation power source.

During use, the atrial septostomy device needs to be used in combination with the ablation power supply, a neutral electrode plate etc. The method of use is as follows.

After the atrial septum is punctured, a guide wire is delivered into the left superior pulmonary vein, and then the puncture kit is removed. The core 55 and the sheath 54 are advanced over the guide wire into the left atrium, and the guide wire and the core 55 are removed.

An atrial septostomy device 20 of a suitable size is used. The pusher 60 extends through the loader from a proximal end thereof, and a proximal end of the atrial septostomy device 20 is connected to a distal end of the pusher 56. Then the pusher 56 is withdrawn to make the atrial septostomy device 20 be received in the loader.

A distal end of the loader is connected to a proximal end of the sheath 54, and the pusher 56 is pushed forward to deliver the atrial septostomy device 20 to a distal end of the sheath 54. The system is observed and adjusted until the marker member is located in the atrial septal tissue. Then, the pusher 56 is slowly pushed or the sheath 54 is withdrawn, during which it needs to make sure that the marker member is located in the atrial septal tissue. The expansion portion 23 of the atrial septostomy device 20 is fully expanded to prop the atrial septal tissue at the puncture hole, so as to form a shunt channel of a specific size (determined by ultrasound or DSC).

After confirming that the tissue at the puncture hole fully conforms to the ablation electrodes 60, the proximal end of the pusher 56 is connected to the RF power supply by a wire, the heating parameters are set (such as a power of 50W, and a duration of 30S), and the heating is started.

After the heating is completed, the atrial septostomy device 20 can be retracted into the sheath 54 and removed from the body, and then it is measured whether the diameter of the puncture hole meets the requirement.

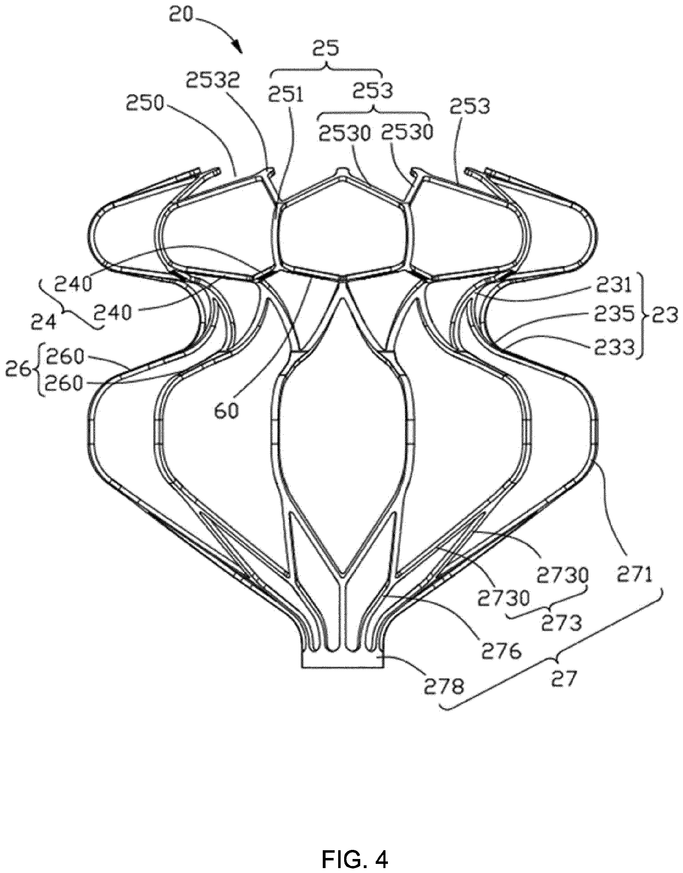

Referring to Fig. 4, FIG. 4 is a schematic structural view of an atrial septostomy device according to a second embodiment of the present application. The structure of the atrial septostomy device according to the second embodiment of the present application is similar to that in the first embodiment, except that in the second embodiment, the ablation electrodes 60 are provided on the first positioning portion 24. Specifically, each first positioning rod 240 is provided with an ablation electrode 60 on a side away from the axis of the support frame 20, and these ablation electrodes 60 are arranged on a circle along the circumference of the support frame 21. When the atrial septostomy device 20 is released in the puncture hole in the atrial septal tissue, the ablation electrodes 60 can ablate the side of the tissue facing the first positioning portion 24 at the puncture hole, to prevent the rebound of the tissue at the puncture hole, and better maintain the shape of the opening.

In other embodiments, the ablation electrodes 60 may also be provided on every other first positioning rod 240 at a side away from the axis of the support frame 21, and these ablation electrodes 60 are arranged on a circle along the circumference of the support frame 21.

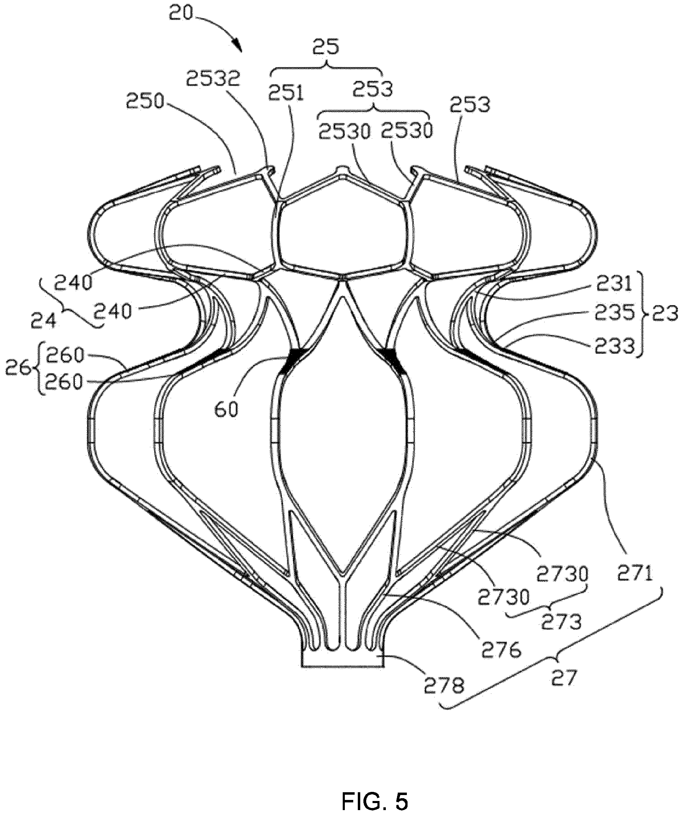

Referring to Fig. 5, FIG. 5 is a schematic structural view of an atrial septostomy device according to a third embodiment of the present application. The structure of the atrial septostomy device according to the third embodiment of the present application is similar to that in the first embodiment, except that in the third embodiment, the ablation electrodes 60 are provided on the second positioning portion 26. Specifically, each second positioning rod 260 is provided with an ablation electrode 60 on a side away from the axis of the support frame 21, and these ablation electrodes 60 are arranged on a circle along the circumference of the support frame 21. When the atrial septostomy device 20 is released in the puncture hole in the atrial septal tissue, the ablation electrode 60 can ablate the side of the tissue facing the second positioning portion 26 at the puncture hole, to prevent the rebound of the tissue at the puncture hole, and better maintain the shape of the opening.

In other embodiments, the ablation electrodes 60 may also be provided on every other second positioning rod 260 on a side away from the axis of the support frame 21, and these ablation electrodes 60 are arranged on a circle along the circumference of the support frame 21.

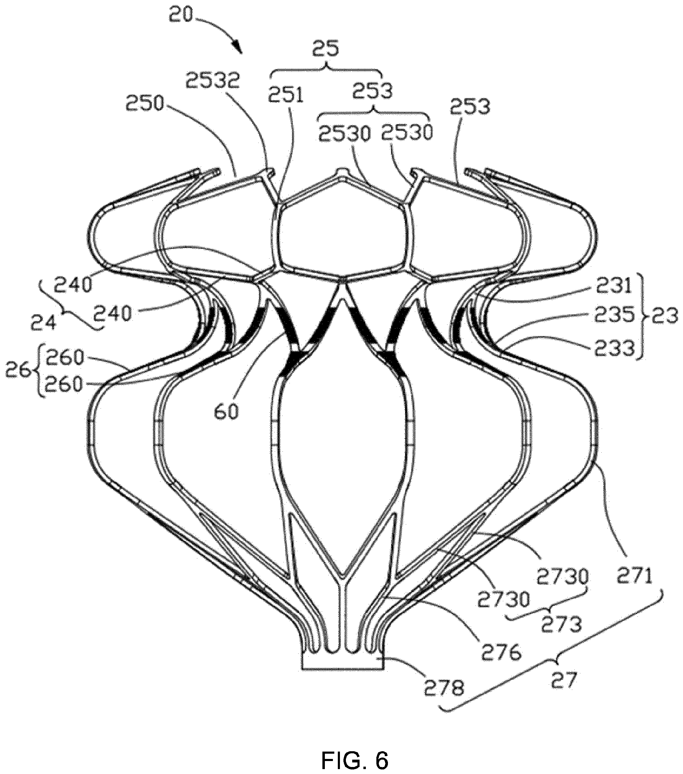

Referring to FIG. 6, FIG. 6 is a schematic structural view of an atrial septostomy device according to a fourth embodiment of the present application. The structure of the atrial septostomy device according to the fourth embodiment of the present application is similar to that in the first embodiment, except that in the fourth embodiment, the ablation electrodes 60 are provided on both the expansion portion 23 and the second positioning portion 26. Specifically, each bar 235 and each second positioning rod 260 is provided with an ablation electrode 60 on a side away from the axis of the support frame 21, and these ablation electrodes 60 are arrange on two circles along the circumference of the support frame 21. When the atrial septostomy device 20 is released in the puncture hole in the atrial septal tissue, the ablation electrodes 60 can ablate both the tissue inside the puncture hole and the tissue facing the second positioning portion 26, to effectively prevent the rebound of the tissue at the puncture hole, and better maintain the shape of the opening.

In other embodiments, the ablation electrodes 60 may also be provided on both the expansion portion 23 and the first positioning portion 24. Alternatively, the ablation electrodes 60 may be provided on both the first positioning portion 24 and the second positioning portion 26. Alternatively, the ablation electrodes 60 may be provided on the expansion portion 23, the first positioning portion 24 and the second positioning portion 26.

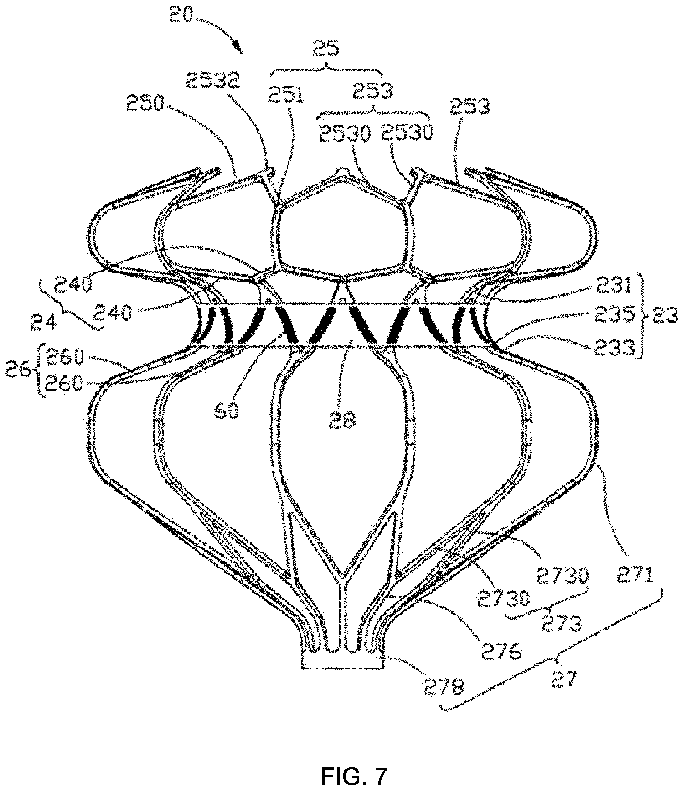

Referring to Fig. 7, FIG. 7 is a schematic structural view of an atrial septostomy device according to a fifth embodiment of the present application. The structure of the atrial septostomy device according to the fifth embodiment of the present application is similar to that in the first embodiment, except that in the fifth embodiment, an insulating film 28 is provided between the support frame 21 and the ablation electrodes 60. Further, the insulating film 28 is located between the ablation electrodes 60 and the expansion portion 23. The insulating film 28 may be, but not limited to, a polytetrafluoroethylene film, a polyurethane film, or a polyimide film, etc. Since the expansion portion 23 and the ablation electrode 60 are isolated by the insulating film 28, the insulating film 28 can isolate the heat conduction between the ablation electrode 60 and the support frame 21, that is, prevent the energy from being transferred to the support frame 21, so as to concentrate the heat on the ablation electrode 60 to ablate the atrial septal tissue, and improve the energy utilization. And also, the insulating film 28 can form an insulating barrier on the side of the ablation electrode 60 facing the blood, to reduce the current density passing through the blood, reduce the heating of blood by the ablation electrode 60, and reduce the risk of thrombosis.

In this embodiment, the insulating film 28 is provided on the expansion portion 23 on an outer wall surface facing the ablation electrode 60. Specifically, the insulating film 28 is attached to the outer wall surface of the expansion portion 23 by stitching or gluing.

The area where the ablation electrode 60 is orthographically projected onto the insulating film 28 is located in the insulating film 28. That is, the area of the orthographic projection of the ablation electrode 60 on the insulating film 28 is less than or equal to the area of the insulating film 28.

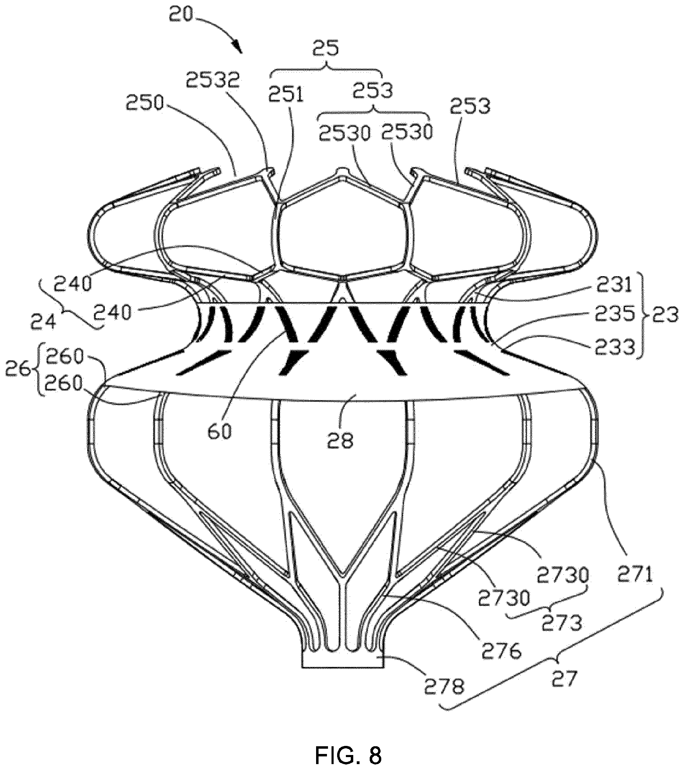

Referring to Fig. 8, FIG. 8 is a schematic structural view of an atrial septostomy device according to a sixth embodiment of the present application. The structure of the atrial septostomy device according to the sixth embodiment of the present application is similar to that in the fourth embodiment, except that in the sixth embodiment, an insulating film 28 is provided between the expansion portion 23 and the second positioning portion 26 of the support frame 21 and the ablation electrodes 60. The insulating film 28 may be, but not limit to, a polytetrafluoroethylene film, a polyurethane film, or a polyimide film, etc. Since the expansion portion 23 and the second positioning portion 26 are isolated from the ablation electrodes 60 by the insulating film 28, the insulating film 28 can isolate the heat conduction between the ablation electrodes 60 and the support frame 21, that is, prevent the energy from being transferred to the support frame 21, so as to concentrate the heat on the ablation electrodes 60 to ablate the atrial septal tissue, and improve the energy utilization. And also, the insulating film 28 can form an insulating barrier on the side of the ablation electrode 60 facing the blood, to reduce the current density passing through the blood, reduce the heating of blood by the ablation electrode 60, and reduce the risk of thrombosis.

In this embodiment, the insulating film 28 is provided on the expansion portion 23 and the second positioning portion 26 on outer wall surfaces thereof facing the ablation electrodes 60. Specifically, the insulating film 28 is attached to the outer wall surfaces of the expansion portion 23 by stitching or gluing.

The insulating film 28 may be a film as a single piece, or two separate films.

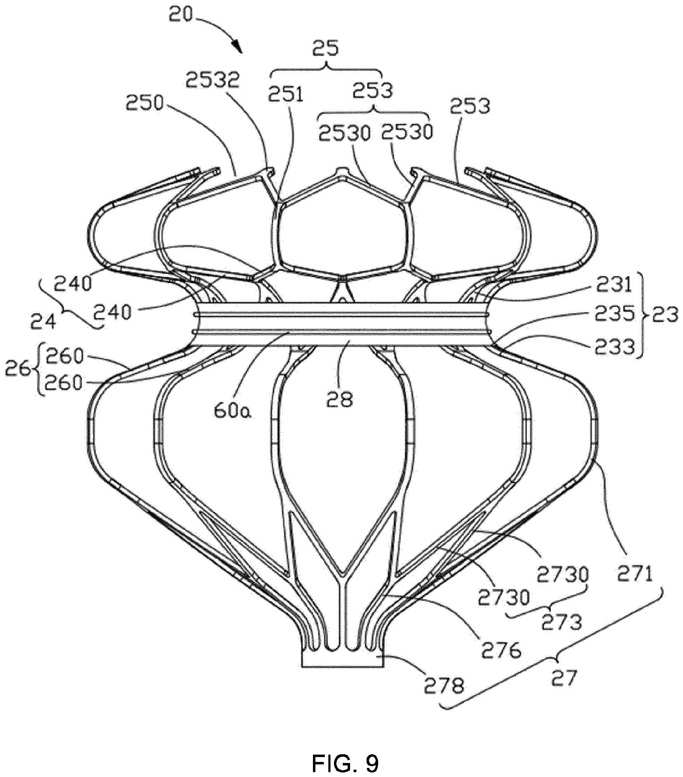

Referring to Fig. 9, FIG. 9 is a schematic structural view of an atrial septostomy device according to a seventh embodiment of the present application. The structure of the atrial septostomy device according to the seventh embodiment of the present application is similar to that in the first embodiment, except that in the seventh embodiment, the ablation electrode 60a is at least one annular electrode arranged on the outer wall of the expansion portion 23, the at least one annular electrode extends along the circumference of the expansion portion 23 for a circle, and the at least one annular electrode is electrically connected to a radio frequency power supply by a flexible wire, wherein the flexible wire is located in the support frame 21. The annular electrode is a continuous ring-shaped, highly elastic, and flexible metal wire, such as a nickel-titanium multi-stranded wire, or a nickel-titanium multi-stranded wire wrapped by a gold spring. The annular electrode can be attached to the support frame 21 by suturing and/or tying.

In this embodiment, the outer wall of the expansion portion 23 is provided with two annular electrodes spaced apart from each other.

An insulating film 28 is provided between the expansion portion 23 and the ablation electrode 60a, so that the expansion portion 23 and the ablation electrode 60a are isolated by the insulating film 28. The insulating film 28 may be, but not limit to, a polytetrafluoroethylene film, a polyurethane film, or a polyimide film, etc.

In other embodiments, the expansion portion 23 is coated with an insulating layer at a side facing the ablation electrodes 60a, for example, a plated Parylene insulating coating. In this way, the ablation electrode 60a and the support frame 21 are insulated from each other.

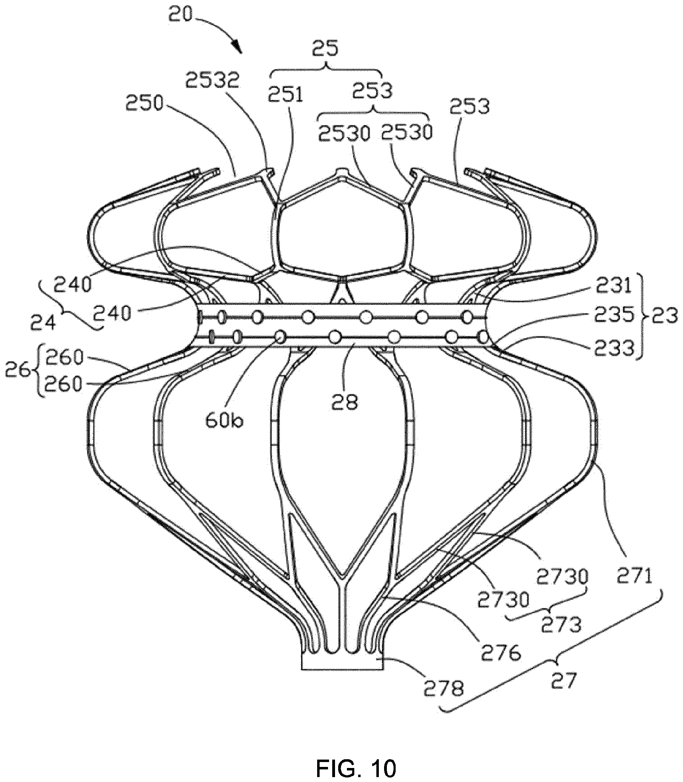

Referring to Fig. 10, FIG. 10 is a schematic structural view of an atrial septostomy device according to an eighth embodiment of the present application. The structure of the atrial septostomy device according to the eighth embodiment of the present application is similar to that in the seventh embodiment, except that in the eighth embodiment, the ablation electrode 60b includes a plurality of spaced dot electrodes. These dot electrodes are arranged on at least one circle along the circumference of the outer wall surface of the support frame 21. Specifically, these dot electrodes are arranged on at least one circle along the circumference of the outer wall surface of the expansion portion 23. Insulation treatment is carried out between the ablation electrode 60b and the support frame 21, by applying an insulating coating on the outer wall surface of the support frame 21 that is in contact with the dot electrodes, or providing an insulating film 28 between the ablation electrode 60b and the support frame 21. The insulating coating may be, but not limited to, FEP/ETFE/PFA, etc. The insulating film 28 may be, but not limited to, a polytetrafluoroethylene film, a polyurethane film, or a polyimide film, etc.

In this embodiment, these dot electrodes are connected in series by a flexible wire and arranged on two circles on the outer wall surface of the expansion portion 23. The flexible wire is electrically connected to a radio frequency power supply.

When the atrial septostomy system in this embodiment is used, it can be used in combination with a loader, a sheath, a core, a conductive pusher, a radio frequency power supply, and a power line. The specific process and method of use are the same as those in the first embodiment, and the details will not be repeated here again.

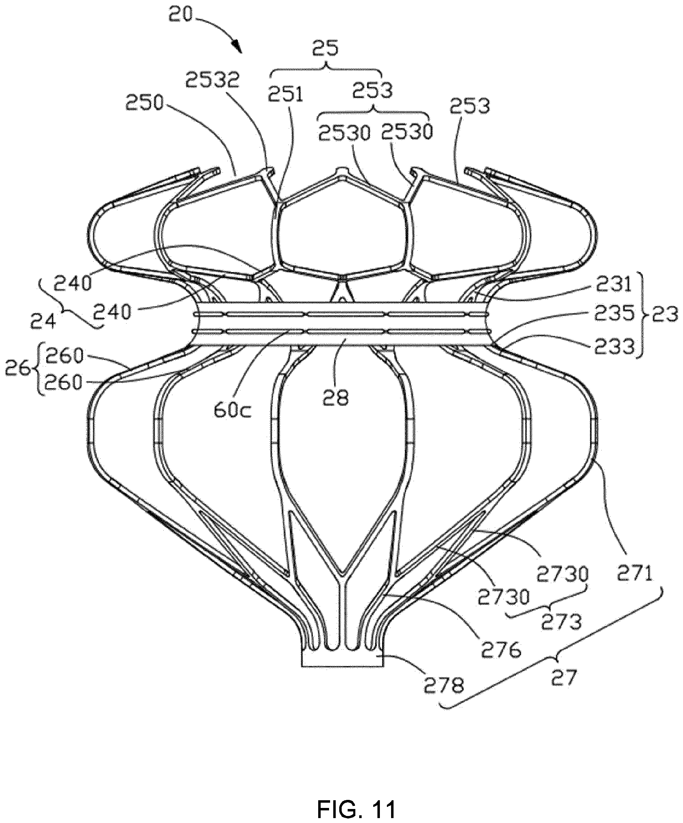

Referring to Fig. 11, FIG. 11 is a schematic structural view of an atrial septostomy device according to a ninth embodiment of the present application. The structure of the atrial septostomy device according to the ninth embodiment of the present application is similar to that in the seventh embodiment, except that in the ninth embodiment, the ablation electrode 60c includes two discontinuous electrode rings arranged along the circumference on the outer wall of the support frame 21. The discontinuous electrode rings are electrically insulated from the support frame 21. Specifically, the two discontinuous electrode rings are provided on the outer wall surface of the expansion portion 23, and an insulating film 28 is provided between the electrode rings and the expansion portion 23. The discontinuous electrode rings are connected in series by a flexible wire and then electrically connected to a radio frequency power supply.

In other embodiments, the ablation electrode 60c may be a single discontinuous electrode ring arranged along the circumference on the outer wall of the expansion portion 23. The single discontinuous electrode ring is connected to an output terminal of a radio frequency power supply by a flexible wire.

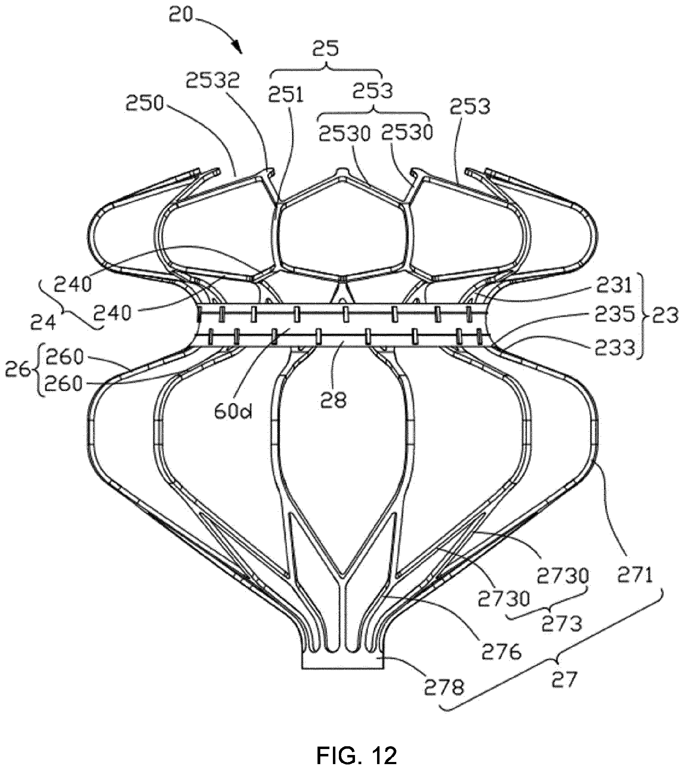

Referring to Fig. 12, FIG. 12 is a schematic structural view of an atrial septostomy device according to a tenth embodiment of the present application. The structure of the atrial septostomy device according to the tenth embodiment of the present application is similar to that in the seventh embodiment, except that in the tenth embodiment, the ablation electrode 60d includes a plurality of spaced rod electrodes. These rod electrodes are arranged on at least one circle along the circumference of the outer wall surface of the support frame 21. Specifically, these rod electrodes are arranged on at least one circle along the circumference of the outer wall surface of the expansion portion 23. Insulation treatment is carried out between the ablation electrode 60d and the support frame 21, by applying an insulating coating on the outer wall surface of the expansion portion 23 that is in contact with the rod electrodes, or providing an insulating film 28 between the ablation electrode 60d and the expansion portion 23. The insulating coating may be, but not limited to, FEP/ETFE/PFA, etc. The insulating film 28 may be, but not limited to, a polytetrafluoroethylene film, a polyurethane film, or a polyimide film, etc.

In this embodiment, these rod electrodes are connected in series by a flexible wire and arranged on two circles on the outer wall surface of the expansion portion 23. The flexible wire is electrically connected to an output terminal of a radio frequency power supply.

In other embodiments, the support frame 21 is made of a conductive material, the ablation electrode is formed by the part of the support frame that does not undergo insulation treatment thereon, and the remaining part of the outer surface of the support frame 21 other than the ablation electrode is coated with an insulating coating or fixed with an insulating sleeve. Preferably, the surface serving as the ablation electrode is provided with a gold-plated layer or a platinum-plated layer thereon. The gold-plated layer or the platinum-plated layer can not only be used as an imaging mark, making it easier for an operator to observe the position of the ablation electrode, but also improve the conductivity of the ablation electrode.

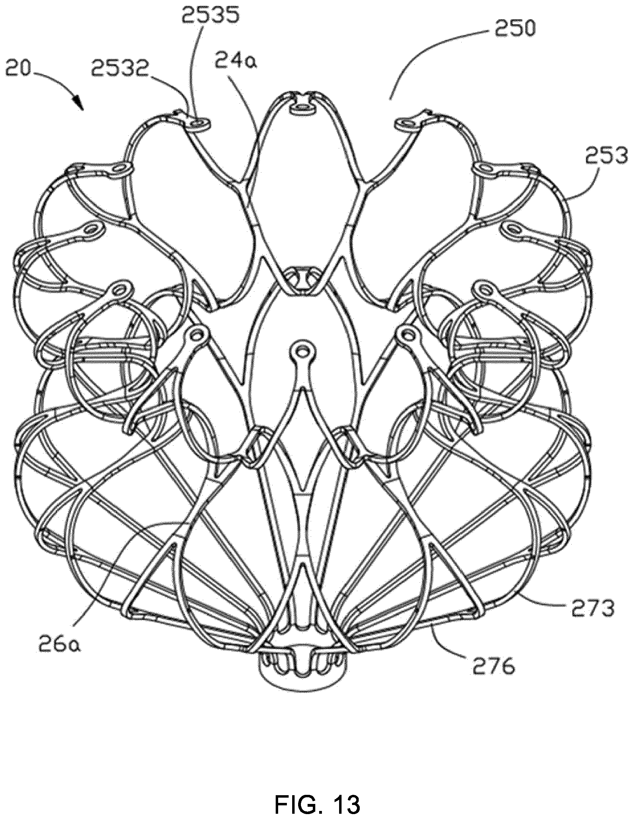

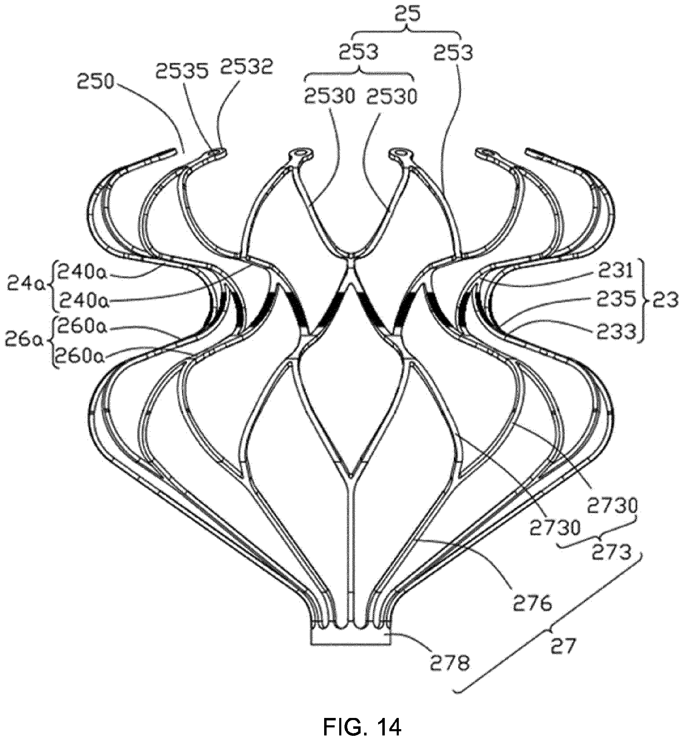

Referring to FIGs. 13 and 14, FIG. 13 is a schematic structural view of an atrial septostomy device according to an eleventh embodiment of the present application, and FIG. 14 is a schematic side view of the atrial septostomy device shown in FIG. 13. The structure of the atrial septostomy device according to the eleventh embodiment of the present application is similar to that in the first embodiment, except that the atrial septostomy device provided in the eleventh embodiment is the atrial septostomy device provided in the first embodiment with the first connecting rods 251 and the second connecting rods 271 are omitted. The structure is specifically described as follows.

The atrial septostomy device provided in the eleventh embodiment includes an expansion portion 23, an extension portion 25 provided at a distal end of the expansion portion 23, and a connecting portion 27 disposed at a proximal end of the expansion portion 23. The extension portion 25 and the expansion portion 23 are connected by a first positioning portion 24a, and the connecting portion 27 and the expansion portion 23 are connected by a second positioning portion 26a. The structure of the expansion portion 23 is the same as that in the first embodiment, and the details will not be repeated here again.

The first positioning portion 24a includes a plurality of first positioning rods 240a, and the plurality of first positioning rods 240a respectively correspond to the plurality of peaks 231. A proximal end of each first positioning rod 240a is connected to the corresponding peak 231, and a distal end of the first positioning rod 240a extends inclinedly distally in a direction away from the axis of the support frame 20. The extension portion 25 includes a plurality of extension members 253 provided at distal ends of the first positioning portion 24a, and the plurality of extension members 253 respectively corresponds to the plurality of first positioning rods 240a. A proximal end of each extension member 253 is connected to the distal end of the corresponding first positioning rod 240a. The plurality of extension members 253 are arranged along the circumferential direction of the support frame 21 to form the extension portion 25. Each extension member 253 includes two branch rods 2530 bifurcated from a radially outermost end of the corresponding first positioning rod 240a. The two branch rods 2530 are bent inclinedly in a direction away from the expansion portion 23. Distal ends of two approaching branch rods 2530 of two adjacent extension members 253 intersect to form an intersected portion 2532. The plurality of intersected portions 2532 extend in the radial direction towards the axis of the support frame 20 to form a converged portion 250. A distal end of each intersected portion 2532 is smoothed. Specifically, the distal end of each intersected portion 2532 is designed as a circular sheet. Since the converged portion 250 extends towards the axis of the support frame 20 and the distal end of each intersected portion 2532 is designed as a circular sheet, the atrial septostomy device 20 is unlikely to damage the important myocardial tissue and is safe and reliable during the operation.

The distal end of the extension member 253 of the extension portion 25 is provided with at least one circle of marker members. Specifically, the distal end of the extension member 253 of the extension portion 25 is provided with at least one circle of marker members centered on the axis of the support frame 20 by inlay or hot pressing, to facilitate the implantation of the atrial septostomy device. In this embodiment, each intersected portion 2532 is provided with a mounting hole 2535, and each mounting hole 2535 is provided with a marker member. Specifically, each mounting hole 2535 is provided with a marker member inlaid therein, and the marker members on the plurality of intersected portions 2532 define a circle. The marker members can be made of gold, platinum, tantalum etc.

In other embodiments, at least one circle of flexible marker wire is provided at the distal ends of the extension members 253 of the extension portion 25, and the marker wire is fixed by winding, inlay, or hot pressing.

The second positioning portion 26a includes a plurality of second positioning rods 260a, and the plurality of second positioning rods 260a respectively corresponds to the plurality of valleys 233. A distal end of each second positioning rod 260a is connected to the corresponding valley 233, and a proximal end of each second positioning rod 260a extends inclinedly proximally in a direction away from the axis of the support frame 20. The connecting portion 27 includes a plurality of support members 273 provided at a proximal end of the second positioning portion 26a, a plurality of extension rods 276 each provided at a proximal end of a respective support member 273, and a connector 278 provided at proximal ends of the extension rods 276. The plurality of support members 273 respectively correspond to the plurality of second positioning rods 260a, a distal end of each support member 273 is connected to the proximal end of the corresponding second positioning rod 260a, and the plurality of support members 273 are arranged along the circumferential direction of the second positioning portion 26a. Each support member 273 includes two branch support rods 2730 bifurcated from a radially outermost end of the corresponding second positioning rod 260a. The two branch support rods 2730 are bent inclinedly in a direction away from the expansion portion 23. Proximal ends of two approaching second positioning rods 260a of two adjacent support members 273 intersect to form an intersected portion. A proximal end of the intersected portion is closer to the axis of the support frame 20 than the radially outermost end of the corresponding branch support rod 2730. The proximal end of each intersected portion further extends proximally to form the extension rod 276. Proximal ends of the extension rods 276 converge at the connector 278, to form a substantially cage shaped structure. The connector 278 has a cylindrical or elliptical tubular structure, with an axial length about 1 to 3 mm, and a smooth edge without sharp corners. The connector 278 can be fixedly or detachably connected to the delivery mechanism.

When the atrial septostomy system in this embodiment is used, it can be used in combination with a loader, a sheath, a core, a conductive pusher, a radio frequency power supply, and a power line. The specific process and method of use are the same as those in the first embodiment, and the details will not be repeated here again. Alternatively, the atrial septostomy system is fixedly connected to a delivery mechanism, and it can be used in combination with a radio frequency power supply, and a power line during use.

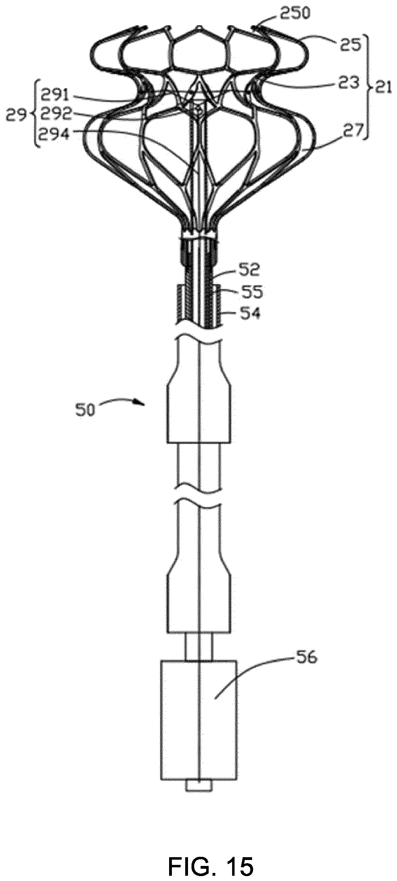

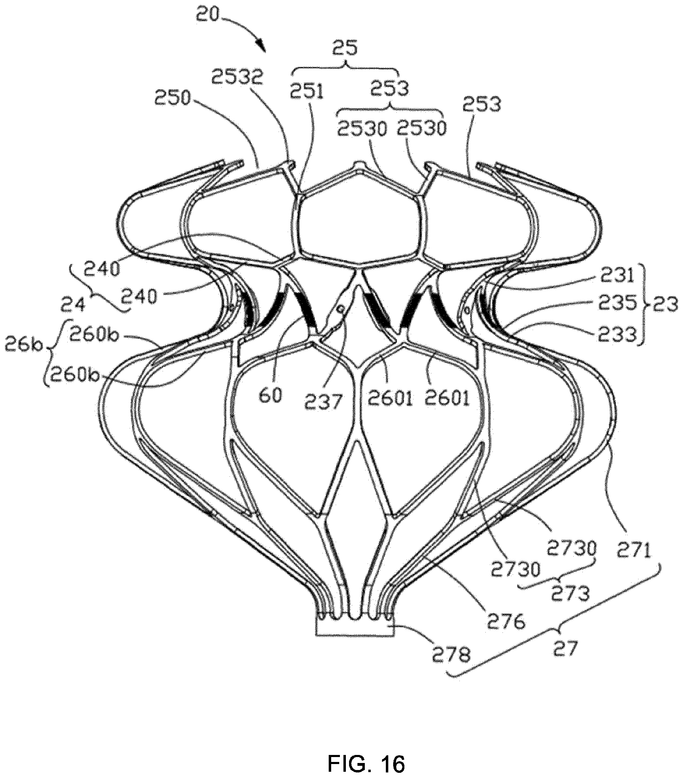

Referring to FIGs. 15 and 16, FIG. 15 is a schematic structural view of an atrial septostomy system according to a twelfth embodiment of the present application, and FIG. 16 is a schematic structural view of an atrial septostomy device shown in FIG. 15. The structure of the atrial septostomy device according to the twelfth embodiment of the present application is similar to that in the first embodiment, except that second positioning portion 26b in the atrial septostomy device provided in the twelfth embodiment is different from that in the first embodiment, and further a size control mechanism 70 is provided in the twelfth embodiment. The structure is specifically described as follows.

The atrial septostomy device provided in the twelfth embodiment includes an expansion portion 23, an extension portion 25 provided at a distal end of the expansion portion 23, and a connecting portion 27 disposed at a proximal end of the expansion portion 23. The extension portion 25 and the expansion portion 23 are connected by a first positioning portion 24, and the connecting portion 27 and the expansion portion 23 are connected by a second positioning portion 26b. The structure of the expansion portion 23, the structure of the first positioning portion 24, and the structure of the extension portion 25 are the same as those in the first embodiment, and the details will not be repeated here again.

The second positioning portion 26b includes a plurality of positioning members 260b, and the plurality of positioning members 260b respectively correspond to the plurality of valleys 233. A distal end of each positioning member 260b is connected to the corresponding valley 233, and a proximal end of the positioning member 260b extends obliquely proximally in a direction away from the axis of the support frame 20. Each positioning member 260b includes two second positioning rods 2601 bifurcated from the corresponding valley 233. The two second positioning rods 2601 are bent inclinedly in a direction away from the expansion portion 23. Proximal ends of two approaching second positioning rods 2601 of two adjacent positioning members 260b intersect to form an intersected portion. A proximal end of the intersected portion is located farther away from the axis of the support frame 20 than the corresponding valley 233. The connecting portion 27 includes a plurality of second connecting rods 271 respectively corresponding to the intersected portions of the second positioning portion 26b, a plurality of support members 273 each provided at a proximal end of a respective second connecting rod 271, a plurality of extension rods 276 each provided at a proximal end of a respective support member 273, and a connector 278 provided at proximal ends of the extension rods 276. The plurality of second connecting rods 271 are arranged along the circumferential direction of the first positioning portion 26b. A distal end of each second connecting rod 271 is connected to the proximal end of the corresponding intersected portion, a proximal end of the second connecting rod 271 is connected to the corresponding support member 273, and a middle portion of the second connecting rod 271 protrudes away from the axis of the support frame 20 to form an arc-shaped rod. Each support member 273 includes two branch support rods 2730 bent and extending radially from two sides from the proximal end of the corresponding second connecting rod 271 towards the axis of the support frame 20. Proximal ends of two approaching branch support rods 2730 of each two adjacent support members 273 intersect to form an intersected portion. A distal end of the intersected portion is closer to the axis of the support frame 20 than the distal end of the second connecting rod 271, and a proximal end of each intersected portion further extends proximally to form the extension rod 276. Proximal ends of the extension rods 276 converge at the connector 278, to form a substantially cage shaped structure. The connector 278 has a cylindrical or elliptical tubular structure, with an axial length about 1 to 3 mm, and a smooth edge without sharp corners. The connector 278 can be fixedly or detachably connected to the delivery mechanism 50.

The atrial septostomy device 20 further includes a size control mechanism 29. The size control mechanism 29 includes a plurality of control threads 291 and a connecting ring 292 arranged in the internal space of the support frame 21, wherein the plurality of control threads 291 are respectively connected between the bars 235 of the expansion portion 23 and the connecting ring 291, and the diameter of the expansion portion 23 can be controlled by pulling the connecting ring 291 proximally.

A plurality of control holes 237 are provided on the bars 235 of the expansion portion 23, and the plurality of control holes 237 are evenly arranged along the circumference of the expansion portion 23. Specifically, each bar 235 is provided with a control hole 237, and the control holes 237 on the plurality of bars 235 are evenly arranged circumferentially to define a circle. Alternatively, every other bar 235 is provided with a control hole 237, and these control holes 237 are evenly arranged in a circle along the circumference of the expansion portion 23. Each control thread 291 extends through two control holes 237 and is then connected to the connecting ring 292.

Preferably, a plurality of control holes 237 are provided along a circumference of the expansion portion 23 with the smallest diameter, and the plurality of control holes 237 are evenly arranged along the circumference of the expansion portion 23. Specifically, the control hole 237 is provided on the corresponding bar 235 at a position closest to the axis of the atrial septostomy device 20.

In this embodiment, four control holes 237 are provided along the circumference of the expansion portion 23 with the smallest diameter, and the four control holes 237 are evenly arranged along the circumference of the expansion portion 23. Four control threads 291 are provided. Two ends of each control thread 291 extend through two adjacent control holes 237 on the expansion portion 23 from the outside to the inside, and then are connected to the connecting ring 292. At this time, the connecting ring 292 is located in the internal space of the expansion portion 23. Preferably, the connecting ring 292 is located at the axis of the expansion portion 23. That is, each control hole 237 has two control wires 291 extending therethrough, and the ends of all the control thread 291 gather at the axis of the expansion portion 23 and are connected to the connecting ring 292 by knotting, to gather the ends of the control threads 291 on the connecting ring 292.

The size control mechanism 29 also includes a pulling thread 294 connected to the connecting ring 292. The pulling thread 294 can be operated to drive the connecting ring 292 to move along the axis of the support frame 21. Specifically, a proximal end of the pulling thread 294 is connected to the connecting ring 292, and a distal end of the pulling thread 294 extends through the connector 278 and the delivery mechanism 50. The pulling thread 294 can be operated to adjust the diameter of the expansion portion 23 through the connecting ring 292 and the control threads 291. Specifically, When the pulling thread 294 is pulled proximally, the connecting ring 292 is driven to move proximally, and the control threads 291 are pulled straight towards the proximal end. The pulling force from the control threads 291 can drive the bars 235 of the expansion portion 23 to resiliently deform and move closer to the axis of the support frame 20, thereby reducing the diameter of the expansion portion 23. When the pulling force on the pulling thread 294 is released, the pulling force on the bars 235 of the expansion portion 23 from the control threads 291 is released, and the bars 235 resiliently returns to their original state.

In the twelfth embodiment, the atrial septostomy device 20 and the delivery mechanism 50 are fixedly connected. The delivery mechanism 20 is fixed to the pushing member 52 by gluing or welding. Before use, the atrial septostomy device 20 is received at the distal end of the sheath 54. In use, the atrial septostomy device is used in combination with the pushing member 52, the sheath 54, the core 55, the pusher 56, an ablation power supply, a power line, and a neutral electrode plate, etc. The method of use is described as follows.

After the atrial septum is punctured, a guide wire is delivered into the left superior pulmonary vein, and then the puncture kit is removed. A passage is established by the guide wire, and the distal end of the sheath 54 is advanced to a position for atrial septostomy. Then the sheath 54 is slowly withdrawn, during which it needs to make sure that the distal end of the sheath 54 is in the left atrium, and the first positioning portion 24 and extension portion 25 of the atrial septostomy device are fully expanded. The various components are kept to have no relative movement, and then the sheath 54 is pulled backward to allow the first positioning portion 24 to tightly abut against the atrial septum. The expansion portion 23 and pushing member 52 are kept in place, and the sheath 55 is withdrawn. The expansion portion 23 and the second positioning portion 26b are fully expanded and the second positioning portion 26b tightly abuts against the atrial septum. At this time, it can be observed by DSC whether the ablation electrode 60 is fully fitted to the atrial septum.

A control handle is adjusted to adjust the diameter of expansion portion 23, so that the puncture hole reaches a required size, wherein the size adjustment amount is ranged from 3 to 18 mm.

After confirming that the ablation electrode 60 is fully fitted to the tissue at the puncture hole, the proximal end of the pusher 56 is connected to a radio frequency power supply, the heating parameters are set (such as a power of 50W, and a duration of 30S), and the heating is started.

After the heating is completed, the atrial septostomy device 20 is withdrawn into the sheath 54 and removed from the body, and then it is measured whether the diameter of the puncture hole meets the requirement.

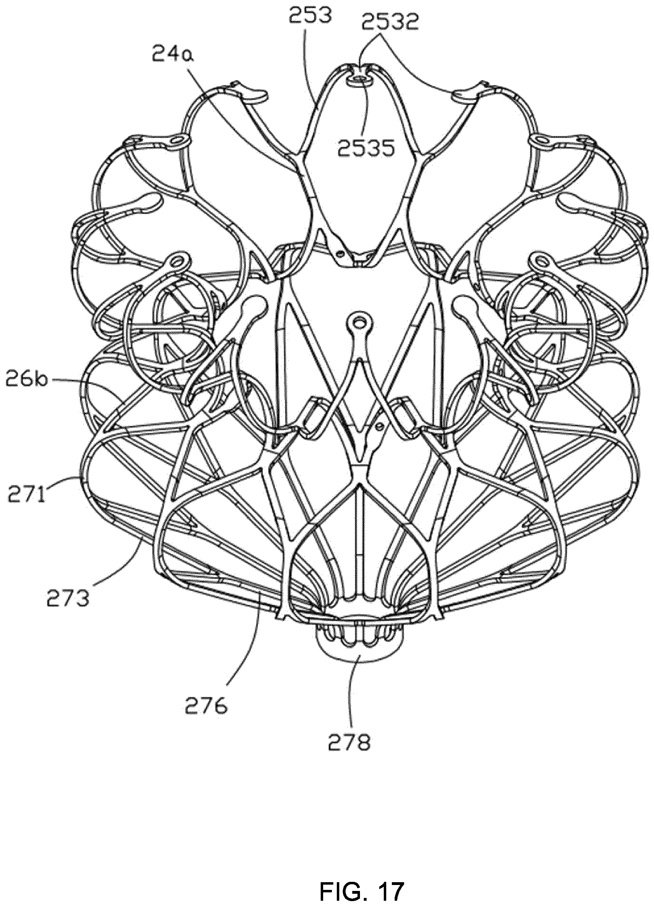

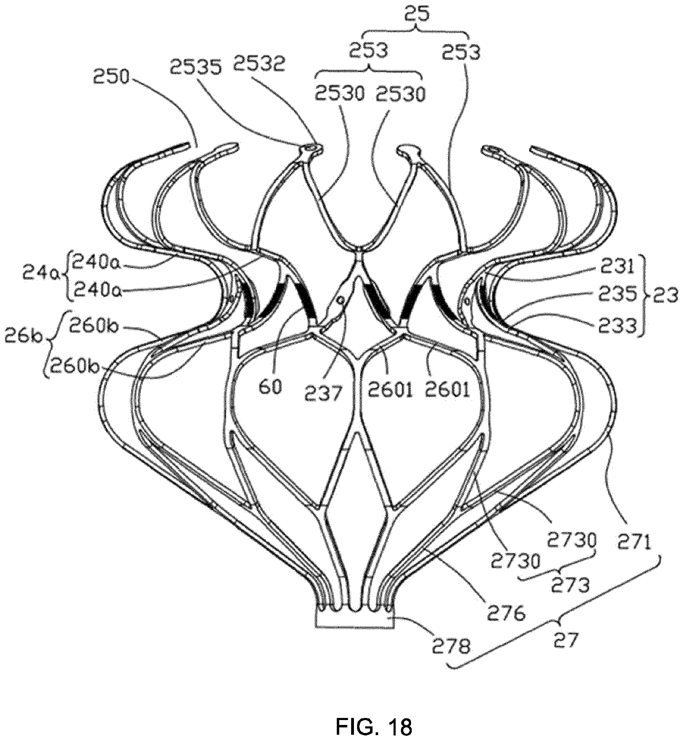

Referring to FIGs. 17 and 18, FIG. 17 is a schematic structural perspective view of an atrial septostomy device according to a thirteenth embodiment of the present application, and FIG. 18 is a schematic side view of the atrial septostomy device shown in FIG. 17. The structure of the atrial septostomy device according to the thirteenth embodiment of the present application is similar to that in the twelfth embodiment, except that the first positioning portion 24a in the atrial septostomy device provided in the thirteenth embodiment is different from that in the twelfth embodiment, and the first connecting rods 251 are omitted. The structure is specifically described as follows.

The atrial septostomy device 20 provided in the thirteenth embodiment includes an expansion portion 23, an extension portion 25 provided at a distal end of the expansion portion 23, and a connecting portion 27 provided at a proximal end of the expansion portion 23. The extension portion 25 and the expansion portion 23 are connected by a first positioning portion 24a, and the connecting portion 27 and the expansion portion 23 are connected by a second positioning portion 26b. The structure of the expansion portion 23, the structure of the second positioning portion 26b, and the structure of the connecting portion 27 are the same as those in the twelfth embodiment. and the details will not be repeated here again.

The first positioning portion 24a includes a plurality of first positioning rods 240a, and the plurality of first positioning rods 240a respectively correspond to the plurality of peaks 231. A proximal end of each first positioning rod 240a is connected to the corresponding peak 231, and a distal end of the first positioning rod 240a extends inclinedly distally in a direction away from the axis of the support frame 20. The extension portion 25 includes a plurality of extension members 253 provided at a distal end of the first positioning portion 24a, and the plurality of extension members 253 respectively correspond to the plurality of first positioning rods 240a. A proximal end of each extension member 253 is connected to the distal end of the corresponding first positioning rod 240a. The plurality of extension members 253 are arranged along the circumferential direction of the first positioning portion 24a, to form the extension portion 25. Each extension member 253 includes two branch rods 2530 bifurcated from a radially outermost end of the corresponding first positioning rod 240a. The two branch rods 2530 are bent inclinedly in a direction away from the expansion portion 23. Distal ends of two approaching branch rods 2530 of two adjacent extension members 253 intersect to form an intersected portion 2532. The plurality of intersected portions 2532 extend towards the axis of the support frame 20 to form a converged portion 250. A distal end of each intersected portion 2532 is smoothed. Specifically, the distal end of each intersected portion 2532 is designed as a circular sheet. Since the converged portion 250 extends towards the axis of the support frame 20 and the distal end of each intersected portion 2532 is designed as a circular sheet, the atrial septostomy device 20 is unlikely to damage the important myocardial tissue and is safe and reliable during the operation.

The distal end of the extension member 253 of the extension portion 25 is provided with at least one circle of marker members centered on the axis of the support frame 20 by inlay or hot pressing, to facilitate the implantation of the atrial septostomy device. In this embodiment, one of every two adjacent intersected portions 2532 is provided with a mounting hole 2535, and each mounting hole 2535 is provided with a marker member by inlaying, and the plurality of marker members on the intersected portions 2532 define a circle. The marker members can be made of gold, platinum, tantalum etc.

When the atrial septostomy system in this embodiment is used, it can be used in combination with a loader, a sheath, a core, a conductive pusher, a radio frequency power supply, and a power line. The specific process and method of use are the same as those in the first embodiment, and the details will not be repeated here again. Alternatively, the atrial septostomy system is fixedly connected to a delivery mechanism, and it can be used in combination with a radio frequency power supply, and a power line during use.

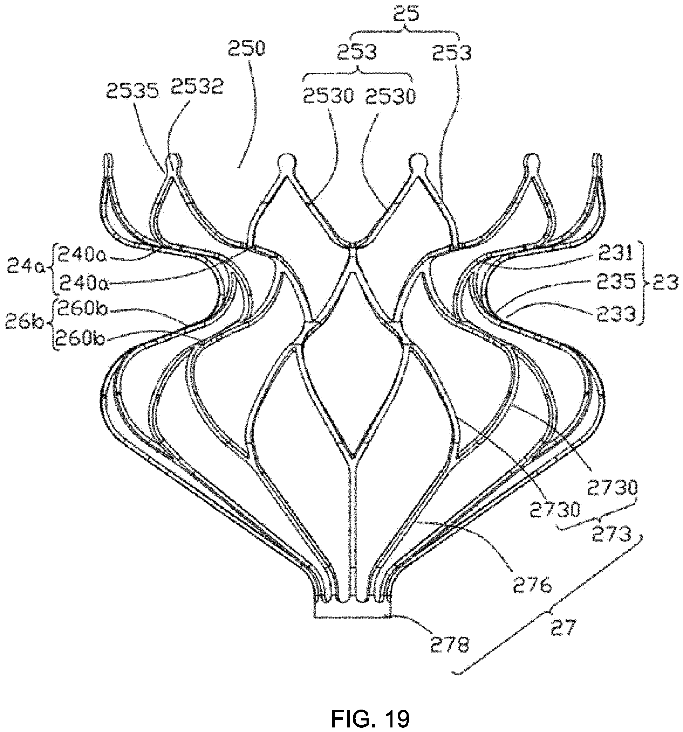

As shown in FIG. 19, the atrial septostomy device 20 provided in the fourteenth embodiment includes an expansion portion 23, an extension portion 25 provided at a distal end of the expansion portion 23, and a connecting portion 27 provided at a proximal end of the expansion portion 23. The extension portion 25 and the expansion portion 23 are connected by a first positioning portion 24a, and the connecting portion 27 and the expansion portion 23 are connected by a second positioning portion 26b. The structure of the expansion portion 23, the structure of the second positioning portion 26b, and the structure of the connecting portion 27 are the same as those in the eleventh embodiment, and the details will not be repeated here again.

This embodiment differs from the eleventh embodiment in that the extension member 253 of the extension portion 25 extends in a direction parallel to the axis of the support frame, and forms a converged portion 250 at the distal end. By means of the extension portion 25 extending in a direction parallel to the axis, it can reduce the radial size of the extension portion 25, and prevent the extension portion 25 as a free end from damaging the myocardial tissue when the atrial septostomy device 20 enters the heart tissue, so as to improve the safety.

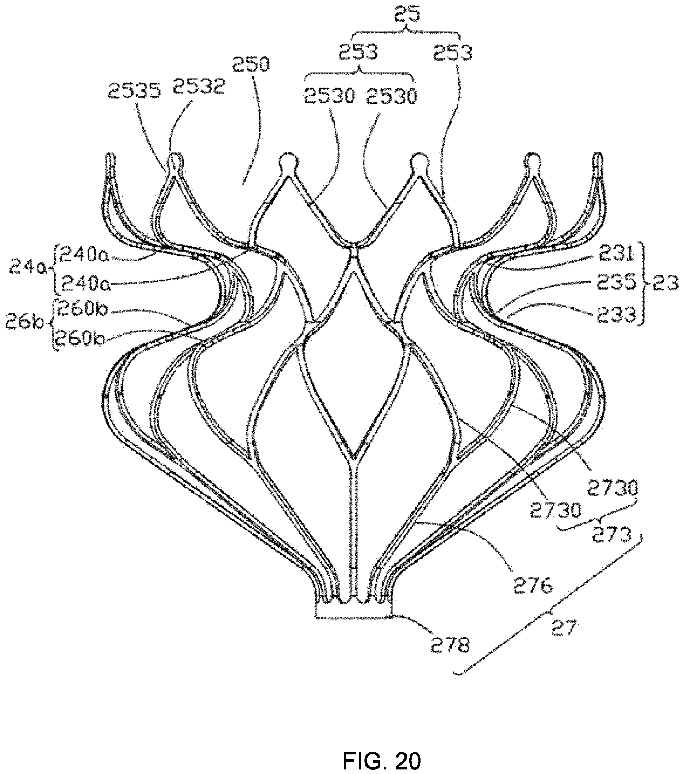

As shown in FIG. 20, The atrial septostomy device 20 provided in the fifteenth embodiment includes an expansion portion 23, an extension portion 25 provided at a distal end of the expansion portion 23, and a connecting portion 27 provided at a proximal end of the expansion portion 23. The extension portion 25 and the expansion portion 23 are connected by a first positioning portion 24a, and the connecting portion 27 and the expansion portion 23 are connected by a second positioning portion 26b. The structure of the expansion portion 23, the structure of the second positioning portion 26b, and the structure of the connecting portion 27 are the same as those in the thirteenth embodiment, and the details will not be repeated here again.

This embodiment differs from the thirteenth embodiment in that the extension member 253 of the extension portion 25 extends in a direction parallel to the axis of the support frame and forms a converged portion 250 at the distal end. By means of the extension portion extending in a direction parallel to the axis, it can reduce the radial size of the extension portion, and prevent the extension portion 25 as a free end from damaging the myocardial tissue when the atrial septostomy device 20 enters the heart tissue, so as to improve the safety.

Described above are embodiments of the present application. It should be noted that those skilled in the art can make various modifications and variations to the embodiments without departing from the spirit and scope of the present application, and these modifications and variations should fall into the scope claimed by the present application.

## Claims

1. An atrial septostomy device, comprising a support frame for propping a puncture hole in an atrial septum, wherein the support frame comprises an expansion portion for expanding the puncture hole, a connecting portion connected to a proximal end of the expansion portion, and an extension portion connected to a distal end of the expansion portion, wherein a distal end of the extension portion has a converged portion that is parallel to an axis of the support frame or extends towards the axis of the support frame; and the support frame is provided with at least one ablation electrode for ablating tissues surrounding the puncture hole.

2. The atrial septostomy device according to claim 1, wherein the expansion portion and the extension portion are connected by a first positioning portion; and the expansion portion and the connecting portion are connected by a second positioning portion, wherein when the expansion portion is located in the puncture hole in the atrial septum, the first positioning portion and the second positioning portion are respectively positioned at two opposite sides of the atrial septum.

3. The atrial septostomy device according to claim 2, wherein the extension portion comprises a plurality of connecting rods provided at a distal end of the first positioning portion, and a plurality of extension members respectively provided at distal ends of the plurality of connecting rods, wherein the plurality of connecting rods are arranged in a circle along a circumferential direction of the first positioning portion, a proximal end of each connecting rod is connected to the first positioning portion, the distal end of each connecting rod is connected to a respective extension member, and the plurality of extension members form the converged portion.

4. The atrial septostomy device according to claim 3, wherein each extension member extends from the distal end of a corresponding connecting rod towards the axis of the support frame, the plurality of extension members are annularly arranged in a circle about the axis of the support frame, each extension member comprises two branch rods bent and extending radially inwardly from two sides towards the axis of the support frame, and distal ends of two approaching branch rods of each two adjacent extension members intersect to form an intersected portion.

5. The atrial septostomy device according to claim 2, wherein the extension portion comprises a plurality of extension members provided at a distal end of the first positioning portion, the first positioning portion comprises a plurality of first positioning rods, the plurality of extension members respectively corresponds to the plurality of first positioning rods, a proximal end of each extension member is connected to a distal end of a corresponding first positioning rod, and the plurality of extension members are arranged along a circumferential direction of the first positioning portion to form the extension portion.

6. The atrial septostomy device according to claim 5, wherein each extension member comprises two branch rods bifurcated from a radially outermost end of the corresponding first positioning rod, the two branch rods are bent inclinedly in a direction away from the expansion portion, distal ends of two approaching branch rods of two adjacent extension members intersect to form an intersected portion, and a plurality of intersected portions extend in parallel to the axis of the support frame or in the radial direction towards the axis of the support frame to form the converged portion.

7. The atrial septostomy device according to claim 3 or 5, wherein a distal end of each extension member is smoothed.

8. The atrial septostomy device according to claim 7, wherein an outer peripheral surface of the distal end of each extension member is formed as an arc surface or have a rounded corner, or the distal end of the extension member is formed as a circular sheet, or a spherical structure.

9. The atrial septostomy device according to claim 4 or 6, wherein an outer peripheral surface of a distal end of each intersected portion is formed as an arc surface or have a rounded corner, or the distal end of the extension member is formed as a circular sheet, or a spherical structure.

10. The atrial septostomy device according to claim 2, wherein
the expansion portion has a wave-shaped annular structure arranged continuously in a circumferential direction for at least one circle, the first positioning portion comprises a plurality of pairs of first positioning rods, the first positioning rods of each pair are bent and extending radially from two sides of each peak of the expansion portion in a direction away from the axis of the support frame, distal ends of two approaching first positioning rods extending from each two adjacent peaks intersect; or
the first positioning portion comprises a plurality of first positioning rods each extending from a respective peak of the wave-shaped annular structure in a direction away from the axis of the support frame.

11. The atrial septostomy device according to claim 10, wherein
the second positioning portion comprises a plurality of second positioning rods each extending from a respective valley of the expansion portion towards a direction away from the axis of the support frame; or
the second positioning portion comprises a plurality of positioning members connected to the proximal end of the expansion portion, wherein each positioning member comprises two second positioning rods bent and extending radially from two sides of a corresponding valley of the expansion portion in a direction away from the axis of the support frame, and proximal ends of two approaching second positioning rods of each two adjacent positioning members intersect.

12. The atrial septostomy device according to claim 10 or 11, further comprising a size control mechanism, wherein the size control mechanism comprises a plurality of control threads and a connecting ring arranged in the internal space of the support frame, wherein the plurality of control threads are respectively connected between bars of the expansion portion and the connecting ring, and a diameter of the expansion portion is controllable by pulling the connecting ring proximally.

13. The atrial septostomy device according to claim 2, wherein
the at least one ablation electrode is provided on one of the expansion portion, the first positioning portion and the second positioning portion;
the at least one ablation electrode comprises a plurality of ablation electrodes provided respectively on two of the expansion portion, the first positioning portion and the second positioning portion; or
the at least one ablation electrode comprises a plurality of ablation electrodes provided respectively on the expansion portion, the first positioning portion and the second positioning portion.

14. The atrial septostomy device according to claim 13, wherein the at least one ablation electrode is formed by a part of the support frame without insulation treatment, and an outer surface of a remaining part of the support frame other than the at least one ablation electrode is coated with an insulating coating or fixed with an insulating sleeve.

15. The atrial septostomy device according to claim 14, wherein a surface of the ablation electrode is provided with a gold-plated layer or a platinum-plated layer thereon.

16. The atrial septostomy device according to claim 13, wherein
the at least one ablation electrode includes an annular electrode, and the annular electrode is arranged for at least one circle continuously or discontinuously along a circumferential direction of the atrial septostomy device on an outer wall surface thereof; or
the at least one ablation electrode includes a plurality of dot electrodes or rod electrodes, and the plurality of dot electrodes or rod electrodes are arranged for at least one circle along the circumferential direction of the atrial septostomy device on the outer wall surface thereof.

17. The atrial septostomy device according to claim 16, wherein
an insulating film is provided between the at least one ablation electrode and the support frame; or
an outer surface of the support frame is coated with an insulating coating corresponding to the at least one ablation electrode.

18. The atrial septostomy device according to claim 2, wherein each of the first positioning portion and the second positioning portion is provided with a positioning surface, a positioning line or a positioning point that contacts the atrial septum, wherein the at least one ablation electrode is provided on the positioning surface, positioning line or positioning point of the first positioning portion or the positioning surface, positioning line or positioning point of the second positioning portion; or
the at least one ablation electrode comprises a plurality of ablation electrodes respectively provided on the positioning surfaces, positioning lines or positioning points of the first positioning portion and the second positioning portion.
